# EUROPEAN PATENT SPECIFICATION

(11) **EP 0 808 455 B1**
(45) Date of publication and mention of the grant of the patent: **21.04.1999**
(21) Application number: 96901887.8
(22) Date of filing: 07.02.1996
(51) Int. Cl.: G01N 30/42, C07K 1/04

(54) **APPLICATION OF COUNTERCURRENT CHROMATOGRAPHY TO THE DECONVOLUTION OF CHEMICAL LIBRARIES**
ANWENDUNG DER GEGENSTROM-CHROMATOGRAPHIE ZUR ENTFALTUNG CHEMISCHER BANKEN
APPLICATION DE LA CHROMATOGRAPHIE A CONTRE-COURANT A LA DECONVOLUTION DE BANQUES DE COMPOSES CHIMIQUES

(30) Priority: 09.02.1995 GB 9502540
(43) Date of publication of application: 26.11.1997
(73) Proprietor: ZENECA LIMITED, London W1Y 6LN (GB)
(72) Inventor: SHUTE, Richard, Eden, Cheshire SK10 3AF (GB); MAIN, Brian, Geoffrey, Cheshire CW11 4HW (GB)
(74) Representative: Phillips, Neil Godfrey Alasdair
(86) International application number: GB9600242
(87) International publication number: WO9624842

(56) References cited:
- EP-A- 0 238 892
- US-A- 5 141 926
- US-A- 5 349 052
- US-A- 5 359 036
- JOURNAL OF CHROMATOGRAPHY, vol. 603, 1992, AMSTERDAM NL, pages 73-81, XP002004011 C. WINGREN ET AL: "supports for liquid-liquid partition chromatography in aqueous two-phase systems: a comparision of Superdex and LiParGel"
- ANGEWANDTE CHEMIE. INTERNATIONAL EDITION, vol. 31, no. 4, 1 April 1992, pages 367-383, XP000325208 JUNG G ET AL: "MULTIPLE PEPTIDE SYNTHESIS METHODS AND THEIR APPLICATIONS"

## Description

This invention relates to methods for separating mixtures of chemical compounds. In particular it relates to the deconvolution of chemical libraries using countercurrent chromatography (CCC).

Available methods for the deconvolution of chemical libraries include iterative resynthesis, affinity selection, positional scanning, self-decipherment or orthogonality, bead selection followed by sequencing, and/or decoding of encoded tags, and solid-liquid adsorption chromatographic methods, including reverse phase high performance liquid chromatography (RP-HPLC). These methods have been reviewed extensively (Gallop, M. A. et al, J. Med. Chem., 1994, 37: 1233-51; Gordon, E. M. et al, J. Med. Chem., 1994, 37, 1385-401; Madden, D. et al, Perpectives in Drug Design, 1994, 2: 269-85; Chabala, J. C. et al, Perpectives in Drug Design, 1994, 2: 305-18; Terrett, N. K. et al, Tetrahedon, 1995, 51: 8135-73; Lowe, G., Chem. Soc. Rev., 1995, 309-17). However the need still exists for further improved methods of library deconvolution.

We have now found that it is possible to partition chemical libraries using CCC so as to identify useful sub-library fractions and individual library members. This is especially pleasing since hitherto CCC has only been applied to the isolation and/or purification of compounds from natural product brews and extracts, antibiotics and peptides [Ito, Y., Crit. Rev. Anal. Chem., 1986, 17, 65-143; Conway, W. D., 1990, 'Countercurrent Chromatography: Apparatus, Theory and Applications', VCH Publishers, New York; Brill, G. M. et al, J. Liq. Chromatog., 1985, 8: 2259-80; Martin, D. G. et al, Am. Lab. (Fairfield, Conn.), 1986, 18th October, 21-26; Farthing, J. E. et al; J. Liq. Chromatog., 1990, 13: 941-50; Lee, S. S.; J. Chromatog., 1994, A, 667: 322-6; Hermanslokke et al, J. Chromatog., 1994, A, 664: 45-53]. Such compounds are present individually against a background of unrelated species.

US-A-5349052 describes fractionating a mixture of polyethylene glycol (PEG)-protein adducts having different degrees of PEG substitution by partitioning the PEG protein adducts in a PEG containing aqueous biphasic system according to the degree of PEG substitution. There is however no suggestion that this method could be used to separate a library of unknown and unrelated low molecular weight chemical compounds.

Therefore in a first aspect of the invention we provide a method for the deconvolution of a chemical library comprising compounds having a molecular weight of less than 1000 daltons, which method comprises separating the library into sub-library fractions by applying countercurrent chromatography (CCC) to the library and collecting sub-library fraction(s); testing the resulting sub-library fraction(s) in a biological assay, test system or screen; and analysing one or more of the sub-library fraction(s) to determine the identity of one or more of the individual library members.

CCC does not require a solid phase and essentially all of the available sample is processed with 100% recovery of all material. This latter facet of CCC is of particular importance in library deconvolution.

Furthermore we have found that CCC separates compounds in a manner unlike other forms of chromatography such as HPLC, hence the chemical library is further characterised and purified.

The method of the invention allows the person of ordinary skill to separate a chemical library into a variety of useful sub-library fractions. The library is partitioned according to parameters useful in drug discovery and design. CCC is, in essence, a form of liquid-liquid partition chromatography, where materials are separated according to their partition coefficients in the particular stationary and mobile phases. Hence, partition coefficients can be measured by CCC (Conway, "Countercurrent Chromatography: Apparatus, Theory and Applications", VCH Publishers, New York; Vallat et al, J. Chromatog., 1990, 504: 411-9). Furthermore, the corrected partition coefficient parameter ClogP, being a useful measure of hydrophobicity/hydrophilicity and often correlated with biological activity, is of importance in the quantitative structure-activity relationships (QSAR) of drug molecules. ClogP also plays a significant role, particularly when corrected for ionisation properties (pKa) of the drug to give logD's, in the delivery of drug to its site of action. Furthermore, whilst ClogP can be positive or negative, there is a tendency for compounds of interest in drug discovery and design to have a ClogP greater than zero, more specifically to have a ClogP between 1 and 4. For log D that preferred range is between 0 and 2. Hence, ClogP and logD are fundamental parameters for consideration during the processes of drug design. Accordingly the present invention is of major relevance to drug discovery and design processes.

Sub-library fractions obtained using the method of the invention may be obtained from any phase during the CCC fractionation to provide any convenient number of sub-library fractions of any convenient size. Such fractions preferably have a ClogP greater than zero, more specifically between about 1 and 4. Their log D is preferably in the range between about 0 and 2.

Any convenient form of CCC such as droplet, centrifugal or planetary CCC may be employed. Planetary or centrifugal coil CCC are preferred.

The chemical library may comprise any convenient number of individual members, for example tens to hundreds to thousands to millions etc., of suitable compounds, for example peptides, peptoids and other oligomeric compounds (cyclic or linear), and template-based smaller molecules, for example benzodiazepines, hydantoins, biaryls, polycyclic compounds (eg. naphthalenes, phenothiazines, acridines, steroids etc.), carbohydrate and amino acids derivatives, dihydropyridines, benzhydryls and heterocycles (eg. triazines, indoles etc.). The numbers quoted and the types of compounds listed are illustrative, but not limiting. For ease of analysis the chemical library may be provided as any convenient number of sub-library mixtures. These may typically contain about 10-1000, such as up to about 100 or up to about 50 library compounds.

It will be appreciated that the method of the invention may be used to separate all or any convenient part of the chemical library into sub-library fractions. By way of example the compounds in a given library are divided between a number of individual library mixtures. One or more of the library mixtures are used in the method of the invention, either prior to or after biological screening of the library.

Preferred compounds are chemical compounds of low molecular weight and potential therapeutic agents. They are for example of less than 800 daltons, such as less than 600 or 400 daltons.

Conveniently the identity of sub-library fractions and individual compounds is determined using conventional analytical techniques such as mass spectrometry (MS) techniques or nuclear magentic resonance (NMR) techniques.

By way of non-limiting example, a library comprises 2,000 compounds distributed evenly between 20 fractions or samples. These 20 samples (A to T) are tested in biological screens relevant to drug discovery and sample Q may be identified as active. A portion of sample Q is then submitted to CCC and fractionated into a suitable number of fractions using a solvent system selected by any person skilled in the art. The number of fractions into which a chemical library sample can be fractionated is not limiting, but is preferably in the range 10 to 30. Then, if the chosen solvent system comprises an aqueous stationary phase the earliest fractions from the CCC instrument will contain those members of the library present in sample Q which possess the highest ClogP. Furthermore, it is possible at any time during the CCC fractionation to switch the phase-mode from stationary-aqueous to stationary-organic. This allows library members of low ClogP to be fractionated during the same experiment and yields a set of fractions wherein all the library members contained in sample Q are distributed across a spectrum of ClogP. Similarly, if the chosen solvent system comprises an organic stationary phase, the earliest fractions eluted from the CCC instrument will contain those members of the library present in sample Q which possess the lowest ClogP. Again it is posible at any time during the CCC fractionation to switch the phase-mode from stationary-organic to stationary-aqueous and allow library members of high ClogP to be fractionated during the same experiment again yielding a set of fractions wherein all the library members contained in sample Q are distributed across a spectrum of ClogP. Furthermore, sample Q may be fractionated twice or more times using CCC, but utilising different solvent systems and different phase-modes, both stationary-aqueous and stationary-organic, to give sets of fractions containing the library members present in sample Q, but wherein those library members are distributed in a different fashion according to C'logP, wherein C'logP is the corrected partition coefficient for the particular solvent system and is different from ClogP.

The selection of suitable liquid phases for use in the method of the invention will be apparent to the skilled scientist. A particular solvent system comprises isohexane:ethyl acetate:methanol:water. Examples of convenient proportions include for example 1:1:1:1 or 1:2:1:2 or 2:1:2:1. The solvent system may optionally containing pH-modifying additives, such as for example triethylamine.

The sub-library fractions may contain any convenient number of compounds, for example between 1 and 20 compounds, but preferably between 1 and 10 compounds.

The resultant sub-library fractions from CCC may be re-tested in the biological screens relevant to drug discovery. Those fractions which were identified as active could then be re-submitted to CCC for further fractionation and re-test, or they may be submitted to a conventional separation technique, for example HPLC, which, if linked to mass spectrometry techniques, for example LC-MS, or to nuclear magnetic resonance techniques, for example LC-NMR, may identify the library member or members contained in that sub-library fraction. If the conventional separation technique were to indicate only a single compound to be present in the active fraction(s), then, by inference, this compound will be the library member responsible for the activity. Hence the library is deconvoluted.

A further aspect of the invention is that analysis of the active sub-library fractions by, for example, LC-MS or LC-NMR may reveal that albeit there is more than one compound in each of the active fractions, there is only a single library member present in all of the active fractions. This member will again, by inference, be the active compound. Hence the library is once more deconvoluted.

A further aspect of the invention is the use of CCC to pre-fractionate chemical libraries before testing to remove library members whose partition coefficients may be unacceptable for drug discovery. Hence, by way of example, for the twenty fraction library mentioned above, each fraction is sub-fractionated prior to biological testing by a person of ordinary skill using CCC. Thus, if an aqueous stationary phase is used, then the very earliest fractions eluted using the organic mobile phase will contain the library members possessing the highest ClogP's. These may, if desired, be discarded prior to testing. Similarly, if an organic stationary phase were used, then the very earliest fractions eluted using the aqueous mobile phase will contain the library members possessing the lowest ClogP's. These may also, if desired, be discarded prior to testing. Hence, by this method a chemical library is produced wherein the members possess partition coefficients only within the range preferred for drug discovery. That preferred range is ClogP between about 1 and 4, or logD between about 0 and 2.

Whilst we do not wish to be limited by theoretical considerations, as outlined above the method of the invention may be used to separate library sub-mixtures until individual compounds are identified. Alternatively sub-library fractions may be further separated via conventional techniques such as HPLC.

The invention will now be illustrated, but not limited by reference to the following Examples:

### Example 1

### Countercurrent chromatographic separation of a tris amino triazine library:

A library was prepared based on a sym-triazine nucleus, using the 'mix and divide' process (Furka et al, Int.J.Pept.Prot.Res., 1991, 37, 487-493) and using three amines in position 2, three amines in position 4, and a further three amines in position 6 to provide a 3*3*3 library of three mixtures, each containing nine components.

The mixtures were examined by HPLC / mass spectrometry to confirm the presence of the expected components, and then one of the mixtures was subjected to separation by CCC using a planetary coil countercurrent chromatograph.

20 mg of the mixture was purified on a 60ml coil. The solvent system was isohexane:ethyl acetate:methanol:water:triethylamine in a ratio of 2:1:2:1:0.06. The coil was first filled with upper, organic phase and the lower, aqueous phase was pumped at 4mVminute (head to tail) until equilibrium was reached; the coil rotation speed was 900rpm. The sample, dissolved in lower phase, was then injected, and pumping continued for 20 minutes Twenty 1ml fractions were collected initially. The direction of coil rotation was then reversed and pumping continued for a further 10 minutes tail to head. Five 2 minute fractions were collected. The coil was then emptied, giving a total of 26 fractions.

Examination of the fractions by HPLC indicated a 3-component mixture in fractions 3 and 4, 1 component in fractions 5 and 6, a 2-component mixture in fraction 7, 1 component in fractions 8 and 9, 2 components in fraction 10, 1 component in fractions 11-14, a 4-component mixture in fractions 19-25, and a mixture in fraction 26 containing predominately 2 components. Thus, after evaporation of solvent, the chromatography afforded three of the nine components pure, three 2-component mixtures, one 3-, and one 4-component mixture.

### Example 2

### Countercurrent chromatographic separation of a cysteine derived library:

A library was prepared based on a cysteine template. One part of this library was prepared by taking 1.5 grams of 1-[N-(tert-butoxycarbonyl)-L-cysteinyl]morpholine (5 mmol) in acetone (80ml) with excess anhydrous potassium carbonate (1.38 grams, 10 mmol) and adding 0.1 equivalents of nine alkylating reagents (0.5 mmol, see list below) at 10 minute intervals. After several hours stirring an excess of a suitable 'capping' reagent was added, in this case ethyl bromide (272 mg, 2.5 mmol), and stirring continued for several more hours. Filtration to remove the inorganic salts and evaporation to remove solvent and excess 'capping' reagent afforded the library mixture. The presence of the ten products was confirmed by HPLC analysis and then the mixture was subjected to separation by CCC using a planetary coil countercurrent chromatograph.

60 mg of the mixture was purified on a 60ml coil. The solvent system was prepared from isohexane:ethyl acetate:methanol:water in a ratio of 1:1:1:1 and allowed to equilibrate. The coil was first filled with lower 'aqueous' phase and the upper phase pumped at 4ml/minute (tail to head) until equilibrium was reached at a coil rotation speed of 900rpm. The sample, dissolved in upper phase, was injected into the solvent line and pumping continued for 30 minutes with fraction collection (1 x 12ml, 2 x 4ml, 6 x 8ml, 1 x 12ml, 2 x 20ml). The coil was then emptied, giving a total of 13 fractions.

Examination of the fractions by HPLC gave the folowing results:
fraction 1 nothing
fraction 2 component A
fraction 3 components A & B plus some of C & D
fraction 4 components C & D plus some of B & E
fraction 5 components D, E, & F
fraction 6 components E & F
fraction 7 component F
fraction 8 nothing
fraction 9 some of component G
fraction 10 components G & H
fraction 11 component G plus some of H
fraction 12 component J
fraction 13 component I plus some of J

Thus the chromatography afforded four fractions containing only one compound and seven other fractions containing up to four components.

### Alkylating Reagents:

A 3,4-dichlorobenzyl bromide
B 3,3-dimethylallyl bromide
C benzyl bromoacetate
D methyl 3-(bromomethyl)benzoate
E bromoacetophenone
F 2-(bromomethyl)benzonitrile
G bromoacetonitrile
H 2-methoxyethyl bromide
I bromoacetamide
J ethyl bromide

## Claims

1. A method for the deconvolution of a chemical library comprising compounds having a molecular weight of less than 1000 daltons, which method comprises separating the library into sub-library fractions by applying countercurrent chromatography (CCC) to the library and collecting sub-library fraction(s); testing the resulting sub-library fraction(s) in a biological assay, test system or screen; and analysing one or more of the sub-library fraction(s) to determine the identity of one or more of the individual library members.

2. A method as claimed in claim 1 wherein CCC is used to provide fraction(s) having a ClogP of greater than 0.

3. A method as claimed in claim 1 or claim 2 wherein CCC is used to provide fraction(s) having a ClogP of between 1 and 4.

4. A method as claimed in claim 1 or claim 2 wherein CCC is used to provide fraction(s) having a logD of greater than 0.

5. A method as claimed in claim 4 wherein CCC is used to provide fraction(s) having a logD of between 0 and 2.

6. A method as claimed in any one of the previous claims wherein the chemical library to be separated is provided as sub-library mixtures, CCC is applied to one or more of the mixtures and sub-library fraction(s) are collected.

7. A method as claimed in claim 6 wherein sub-library fraction(s) are separated to provide further sub-library mixtures, CCC is applied to one or more of these and sub-library fraction(s) are collected, said method being optionally repeated at least once.

8. A method according to any one of the previous claims wherein individual member(s) of the library are identified using mass spectrometry or nuclear magnetic resonance spectroscopy.

9. A method as claimed in claim 8 wherein liquid chromatography is linked to the mass spectrometry or nuclear magnetic resonance spectroscopy.

## Patentansprüche

1. Verfahren zur Entfaltung einer chemischen Bank, umfassend Verbindungen, die ein Molekulargewicht von weniger als 1000 Dalton haben, wobei das Verfahren das Trennen der Bank in Sub-Bankfraktionen durch Anwenden der Gegenstrom-Chromatographie (CCC) auf die Bank und das Sammeln einer (von) Sub-Bankfraktion(en), das Testen der erhaltenen Sub-Bankfraktion(en) in einem biologischen Test, Testsystem oder Suchtest sowie das Analysieren von einer oder mehreren Sub-Bankfraktion(en) umfaßt, um die Identität von einem oder mehreren einzelnen Bankmitgliedern zu bestimmen.

2. Verfahren nach Anspruch 1, wobei die CCC zur Bereitstellung einer (von) Fraktion(en) verwendet wird, die einen ClogP von größer als 0 hat (haben).

3. Verfahren nach Anspruch 1 oder Anspruch 2, wobei die CCC zur Bereitstellung einer (von) Fraktion(en) verwendet wird, die einen ClogP zwischen 1 und 4 hat (haben).

4. Verfahren nach Anspruch 1 oder Anspruch 2, wobei die CCC zur Bereitstellung einer (von) Fraktion(en) verwendet wird, die einen logD von größer als 0 hat (haben).

5. Verfahren nach Anspruch 4, wobei die CCC zur Bereitstellung einer (von) Fraktion(en) verwendet wird, die einen logD zwischen 0 und 2 hat (haben).

6. Verfahren nach einem der vorstehenden Ansprüche, wobei die zu trennende chemische Bank als Sub-Bankgemische bereitgestellt wird, die CCC bei einem oder mehreren der Gemische angewendet wird und (eine) Sub-Bankfraktion(en) gesammelt wird (werden).

7. Verfahren nach Anspruch 6, wobei die Sub-Bankfraktion(en) getrennt wird (werden), so daß weitere Sub-Bankgemische bereitgestellt werden, CCC auf eines oder mehrere davon angewendet wird und (eine) Sub-Bankfraktion(en) gesammelt wird (werden), wobei das Verfahren gegebenenfalls mindestens einmal wiederholt wird.

8. Verfahren nach einem der vorstehenden Ansprüche, wobei ein einzelnes (einzelne) Mitglied(er) der Bank unter Verwendung von Massenspektrometrie oder magnetischer Kernresonanzspektroskopie identifiziert wird (werden).

9. Verfahren nach Anspruch 8, wobei Flüssigchromatographie mit der Massenspektrometrie oder magnetischen Kernresonanzspektroskopie verbunden wird.

## Revendications

1. Procédé pour la déconvolution d'une banque chimique comprenant des composés ayant une masse moléculaire inférieure à 1 000 daltons, lequel procédé comprend la séparation de la banque en fractions de sous-banques par application de la chromatographie à contre-courant (CCC) à la banque et récupération d'une fraction ou de fractions de sous-banques, le test de la ou des fractions de sous-banques résultantes dans un dosage, système de test ou criblage biologique, et l'analyse d'une ou plusieurs des fractions de sous-banques pour déterminer l'identité d'un ou plusieurs des éléments de banque individuels.

2. Procédé selon la revendication 1, où la CCC est utilisée pour donner une ou des fractions ayant un ClogP supérieur à 0.

3. Procédé selon la revendication 1 ou la revendication 2, où la CCC est utilisée pour donner une ou des fractions ayant un ClogP situé entre 1 et 4.

4. Procédé selon la revendication 1 ou la revendication 2, où la CCC est utilisée pour donner une ou des fractions ayant un logD supérieur à 0.

5. Procédé selon la revendication 4, où la CCC est utilisée pour donner une ou des fractions ayant un logD situé entre 0 et 2.

6. Procédé selon l'une quelconque des revendications précédentes, où la banque chimique à séparer est fournie sous forme de mélanges de sous-banques, la CCC est appliquée à un ou plusieurs des mélanges et une ou des fractions de sous-banques est ou sont recueillies.

7. Procédé selon la revendication 6, où la ou les fractions de sous-banques est ou sont séparées pour donner des mélanges de sous-banques supplémentaires, la CCC est appliquée à un ou plusieurs de ceux-ci et une ou des fractions de sous-banques est ou sont recueillies, ledit procédé étant éventuellement répété au moins une fois.

8. Procédé selon l'une quelconque des revendications précédentes, où un ou des éléments individuels de la banque est ou sont identifiés par spectrométrie de masse ou spectroscopie de résonance magnétique nucléaire.

9. Procédé selon la revendication 8, où la chromatographie liquide est couplée à la spectrométrie de masse ou à la spectroscopie de résonance magnétique nucléaire.
